(19) Europäisches Patentamt
European Patent Office
Office européen des brevets

(11) Publication number: 0 152 450 B1

(12) EUROPEAN PATENT SPECIFICATION

(45) Date of publication of patent specification: 03.05.89

(51) Int. Cl.$^4$: C 07 C 17/26

(21) Application number: 84903119.0

(22) Date of filing: 30.07.84

(86) International application number: PCT/US84/01194

(87) International publication number: WO 85/01045 14.03.85 Gazette 85/07

# (54) CATALYZED GRIGNARD COUPLING PROCESS.

(30) Priority: 19.08.83 US 524706
30.01.84 US 575131

(43) Date of publication of application:
28.08.85 Bulletin 85/35

(45) Publication of the grant of the patent:
03.05.89 Bulletin 89/18

(84) Designated Contracting States:
CH DE FR GB LI NL

(56) References cited:
US-A-4 124 004

BULLETIN CHEMICAL SOCIETY OF JAPAN, vol. 53, March 1980; IBUKI et al., pp. 821-822

CANADIAN JOURNAL OF CHEMISTRY, vol. 53, 1 Dec 1980; MITCHELL et al., pp. 2584-2587

JOURNAL CHEMICAL SOCIETY, Chem. Comm., 1972; Corriu et al., p. 144

(73) Proprietor: FMC Corporation
2000 Market Street
Philadelphia Pennsylvania 19103 (US)

(72) Inventor: DELMAR, Eric, George
116 Featherbed Lane
Hopewell, NJ 08525 (US)

(74) Representative: Vossius & Partner
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 152 450 B1

## Description

This invention is in the field of chemical processes; more specifically, cross-coupling an aryl Grignard reagent with a halobenzene in the presence of a catalyst to produce a biphenyl compound.

Pyrethroid ester insecticides are of great commercial interest throughout the world. Both the carboxylic acid and alcohol moieties of these esters cover a wide rang of structures. The alcohol, (2-methyl[1,1'-biphenyl]-3-yl)-methanol, affords a series of esters having especially attractive insecticidal efficacy when combined with appropriate carboxylic acids; for example, 3-(2,2-dihaloethenyl)-2,2-dimethylcyclopropanecarboxylic acids and 3-(2-chloro-3,3,3-trifluoro-1-propenyl)-2,2-dimethylcyclopropanecarboxylic acid. Esters of these acids combined with (2-methyl[1,1'-biphenyl]-3-yl)-methanol are described in U.S. 4,214,004 and U.S. 4,238,505, respectively.

Although pyrethroid esters of (2-methyl[1,1'-biphenyl]-3-yl)-methanol have commercial potential, yields of the requisite (2-methyl[1,1'-biphenyl]-3-yl)-methanol from known preparative techniques have been too low to warrant commercialization. For example, U.S. 4,214,004 discloses an overall yield of about 15% beginning with 2-methyl-3-nitrobenzyl alcohol.

According to the multistep synthesis of which the present invention is the key step, the overall yield of (2-methyl[1,1'-biphenyl]-3-yl)-methanol is about 60% utilizing a commercially available 2,6-dihalotoluene, for example, 2,6-dichlorotoluene or 2-bromo-6-chlorotoluene, in the first step to produce a 3-halo-2-methylphenylmagnesium halide, that is, 3-chloro-2-methylphenylmagnesium chloride or bromide, by conventional techniques.

The key to success of the overall process lies in the next step in which the 3-chloro-2-methylphenylmagnesium halide dissolved is cross-coupled with a halobenzene in an inert solvent in the presence of a catalytic amount of catalyst selected from palladium metal or nickel (II) acetylacetonate, producing 3-chloro-2-methyl-[1,1'-biphenyl]. It is this step which is the process of this invention.

Coupling reactions of Grignard reagents are known to lead to mixures of products because of the possibilities for both homo and hetero coupling between the reactants, as well as coupling between the reactants and products. The products from attempted cross-coupling of a Grignard reagent with a halocarbon compound often constitute an intractable mixture with a low yield of the desired cross-coupled product.

When palladium metal is employed as catalyst, the reaction mixture is heterogeneous. Coupling reactions of Grignard reagents are not generally conducted under heterogeneous conditions.

On the basis of the prior art (e.g. as disclosed in J.C.S. Chem. Comm. (1972), p. 144), cross-coupling a 3-chloro-2-methylphenylmagnesium halide with a halobenzene using nickel (II) acetylacetonate as catalyst under homogeneous conditions would seem best carried out by using about 1 mole percent nickel (II) acetylacetonate based on the limiting reagent and perhaps a molar excess of the Grignard reagent. One would expect to obtain a yield of 60—80% 3-halo-2-methyl-[1,1'-biphenyl]. Nothing in the prior art suggests an increase in the yield to about 90% by decreasing the catalyst level one to two orders of magnitude.

The 3-chloro-2-methyl-[1,1'-biphenyl] cross-coupling product can be converted to the desired (2-methyl[1,1'-biphenyl]-3-yl)methanol by various methods. For example, the 3-chloro-2-methyl-[1,1'-biphenyl] can be converted to a second Grignard, a 2-methyl([1,1'-biphenyl]-3-yl)magnesium halide, which then yields the desired (2-methyl[1,1'-biphenyl]-3-yl)-methanol when treated with formaldehyde, processes which are well known in the art.

Although other techniques for preparing the Grignard reagent are known, the 3-chloro-2-methylphenylmagnesium halide is advantageously prepared in an inert solvent. Inert solvents suitable for use in the process include ethers, for example, tetrahydrofuran and diethyl ether, as well as liquid tertiary amines, for example, triethylamine. Tetrahydrofuran is preferred when preparing 3-chloro-2-methylphenylmagnesium chloride, at least two moles of tetrahydrofuran per mole of Grignard reagent.

The so-prepared solution of the Grignard reagent may be added to a warm, stirred mixture of the catalyst in the halobenzene, or the halobenzene may be added to a warm (50° to 80°C preferred), stirred mixture of the catalyst and solution of the Grignard reagent. The rate of addition is not critical, but relatively fast addition, that is, 0.5 to 1.0 hr., is advantageously employed. The reaction generally is completed in a few hours.

Whereas the halobenzene may be bromobenzene or iodobenzene, bromobenzene is preferred. At least a 10% molar excess of halobenzene is preferably used to minimize coupling of the Grignard reagent to the product. The use of iodobenzene leads to more self-coupling to biphenyl, with a corresponding decrease in cross-coupling yield, than if bromobenzene is used.

Generally, a catalytic amount of palladium metal catalyst sufficient to contain about 0.01—1.0, preferably 0.02—0.5, mole percent palladium metal based on the Grignard reagent is sufficient, and larger amounts may actually lead to lower yields of the desired product. Although palladium metal, for example, as commercially available palladium black, can be used, the small amounts required are difficult to weigh and handle, so it is usually desirable to employ palladium metal carried on a solid support such as carbon, alumina, silica, or clay. Palladium metal on either carbon or alumina as solid supports are available commercially and preferred for that reason. At the catalyst levels employed in this invention, catalyst recovery and recycling are not necessary.

If the nickel catalyst is selected, a catalytic amount of nickel (II) acetylacetonate, greater than 0.01 mole

percent, but not more than 0.1 mole percent based on the Grignard reagent is used, with the 0.02—0.04 mole percent range being preferred for maximum yields.

The procedure for preparing the Grignard reagent and conducting the cross-coupling reaction of this invention are illustrated in the following Examples.

## Example 1

Palladium Metal Catalysis

A. *Palladium On Carbon As Catalyst*

Bromobenzene (7.58 g, 48.3 millimoles) and 5% palladium on carbon (0.01 g 4.83 micromole of palladium metal) was stirred under nitrogen at 100°C. To this was added dropwise during a 15 hour period 3-chloro-2-methylphenylmagnesium chloride (4.48 g, 24.2 millimoles) in dry tetrahydrofuran (15 ml), which had been prepared by the method of Mitchell and Yan, *Can. J. Chem., 58,* 2584 (1980). The reaction mixture was stirred for two hours and then analyzed by vapor phase chromatography, which indicated 95.8% of the Grignard reagent had reacted. Of the products which formed, 86.1% was 3-chloro-2-methyl[1,1'-biphenyl], a yield of 82.5% based on the Grignard reagent.

Other similar experiments utilizing palladium on carbon under conditions within the scope of this invention are summarized as follows:

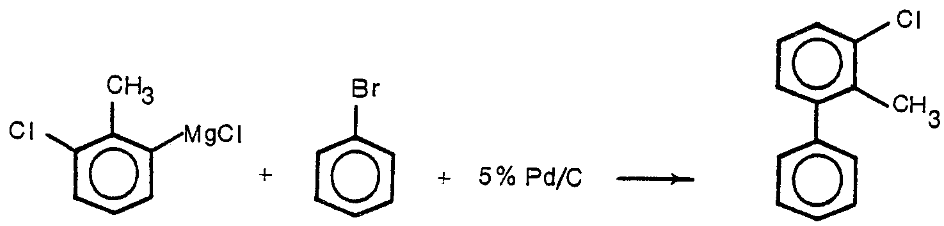


A B C

| Example | Reagents A (mmoles) | Reagents B (mmoles) | Reagents $C^a$ (mole%) | Addition Time (hr) | Reaction[b] Time (hrs) | Reaction[b] Temp (°C) | Yield (%) |
|---|---|---|---|---|---|---|---|
| 2 | 24.2 | 120.8 | 0.5 | 1.5 | 4.0 | 80 | 73.7 |
| 3 | 24.2 | 60.4 | 0.1 | 20.0 | 20.0 | 80 | 77.6 |
| 4 | 24.2 | 48.3 | 0.1 | 2.5 | 2.5 | 100 | 81.3 |
| 5 | 24.2 | 48.3 | 0.01 | 5.5 | 5.5 | 120 | 54.6 |

[a] Mole % based on Grignard reagent.

[b] The reaction time is the total of the addition time, plus the time the reaction was stirred at the reaction temperature after complete addition.

B. *Palladium On Alumina As Catalyst*

Bromobenzene (18.9 g, 120.7 millimoles) and 5% palladium on alumina (0.25 g, 0.12 millimole of palladium metal) was stirred under nitrogen at 80°C. A solution of 3-chloro-2-methylphenylmagnesium chloride 4.48 g, 24.2 millimoles) in dry tetrahydrofuran (15 ml) was added dropwise to the reaction mixture during a 1.25 hour period. After complete addition the mixture was analyzed by vapor phase chromatography. Analysis of the reaction mixture indicated 98% of the Grignard reagent had reacted. Of the products formed, 75.2% was 3-chloro-2-methyl[1,1'-biphenyl], a yield of 73.5% based on the Grignard reagent added.

## Example 2

Nickel (II) Acetylacetonate Catalysis

Under a dry nitrogen atmosphere, a stirred mixture of magnesium turnings (97.3 g, 4.00 moles) in approximately 65 ml of dry tetrahydrofuran was heated to 65°C. To the warm mixture was added one ml of 1,2-dibromoethane. While maintaining the temperature at about 65°C, a solution of 2,6-dichlorotoluene (322 g, 2.00 moles) in dry tetrahydrofuran (600 ml) was added to the mixture during a 45 minute period. After complete addition, the reaction mixture was stirred at 65°C for three hours. Analysis of the reaction

mixture indicated each milliliter of the solution contained 1.698 milliequivalents of 3-chloro-2-methylphenylmagnesium chloride.

Under a dry nitrogen atmosphere, 50 ml of the above-described reaction mixture, containing 0.085 mole 3-chloro-2-methylphenylmagnesium chloride, was stirred and heated at 50°C. Nickel (II) acetylacetonate hydrate (0.00825 g, 0.00003 mole) was added to the mixture. During a one hour period bromobenzene (18.8 g, 0.120 mole) was added dropwise to the warm reaction mixture. After complete addition, the mixture was stirred at 50°C for 23 hours. The mixture was cooled to room temperature and 20.0 ml of n-tetradecane added as an internal standard for GLPC analysis. The resultant mixture was washed with 25 ml of a 0.1 N hydrochloric acid solution. Analysis of the washed mixture by GLPC indicated 20.73% by weight of 3-chloro-2-methyl-[1,1'-biphenyl], an 88.9% yield.

Other similar experiments, arranged in order of increasing amount of catalyst, are summarized as follows:

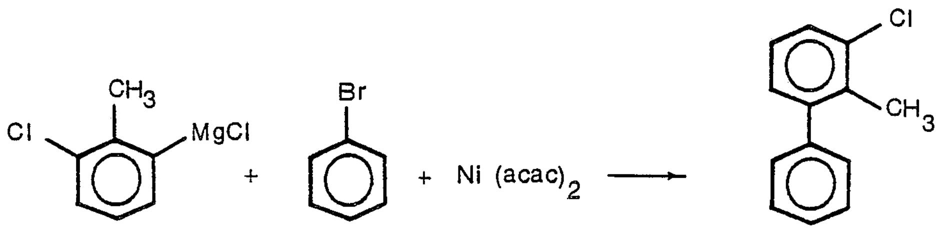


A B C

| Example | Reagents A (moles) | Reagents B (moles) | Reagents C[a] (mole%) | Addition Time (hr) | Reaction[b] Time (hrs) | Reaction[b] Temp (°C) | Yield (%) |
|---|---|---|---|---|---|---|---|
| 2 | 0.10 | 0.11 | 0.01 | 0.17 | 22.0 | 89 | 66.6 |
| 3 | 0.085 | 0.12 | 0.024 | 2.0 | 24.0 | 65 | 84.3 |
| 4 | 0.085 | 0.12 | 0.024 | 1.5 | 24.0 | 80 | 85.0 |
| 5 | 0.085 | 0.12 | 0.024 | 1.5 | 24.0 | 50 | 86.2 |
| 6 | 0.085 | 0.12 | 0.024 | 1.0 | 24.0 | 65 | 86.2 |
| 7[c] | 0.048 | 0.0528 | 0.02 | 0[d] | 9.0 | 80 | 87.8 |
| 8[c] | 1.0 | 1.1 | 0.02 | 0.17 | 3.0 | 25-80 | 88.2 |
| 9[c] | 0.032 | 0.0352 | 0.02 | 0.75 | 4.0 | 80 | 89.2 |
| 1 | 0.085 | 0.12 | 0.035 | 1.0 | 24.0 | 50 | 88.9 |
| 10 | 0.085 | 0.12 | 0.035 | 1.0 | 24.0 | 80 | 84.8 |
| 11 | 0.085 | 0.12 | 0.035 | 1.5 | 24.0 | 65 | 85.1 |
| 12 | 0.085 | 0.12 | 0.035 | 1.5 | 24.0 | 65 | 86.0 |
| 13 | 0.085 | 0.12 | 0.035 | 1.5 | 24.0 | 65 | 86.6 |
| 14 | 0.085 | 0.12 | 0.035 | 2.0 | 24.0 | 80 | 86.5 |
| 15 | 0.085 | 0.12 | 0.035 | 2.0 | 24.0 | 50 | 86.2 |
| 16 | 0.085 | 0.12 | 0.047 | 1.0 | 24.0 | 65 | 85.5 |
| 17 | 0.085 | 0.12 | 0.047 | 1.5 | 24.0 | 50 | 85.7 |
| 18 | 0.085 | 0.12 | 0.047 | 1.5 | 24.0 | 80 | 84.1 |
| 19 | 0.085 | 0.12 | 0.047 | 2.0 | 24.0 | 65 | 82.6 |
| 20[c] | 0.048 | 0.0528 | 0.1 | 0[d] | 5.0 | 90 | 85.4 |
| 21 | 0.10 | 0.15 | 0.11 | 2.25 | 22.0 | 65 | 58.0 |
| 22 | 0.10 | 0.15 | 0.11 | 2.25 | 22.0 | 65 | 57.8 |
| 23 | 0.10 | 0.15 | 0.11 | 2.25 | 22.0 | 65 | 61.8 |
| 24 | 0.10 | 0.20 | 0.2 | 0.5 | 22.0 | 50 | 68.2 |
| 25 | 0.10 | 0.10 | 0.2 | 4.0 | 22.0 | 50 | 43.7 |
| 26 | 0.10 | 0.20 | 0.2 | 4.0 | 22.0 | 80 | 27.3 |
| 27 | 0.10 | 0.10 | 0.2 | 0.5 | 22.0 | 80 | 35.0 |
| 28[c] | 0.093 | 0.466 | 1.0 | 2.0 | 2.0 | 25-45 | 68.4 |
| 29[c] | 0.0246 | 0.246 | 1.0 | 2.3 | 2.3 | 80 | 77.0 |

[a] Mole % based on Grignard reagent.

[b] The reaction time is the total of the addition time, plus the time the reaction was stirred at the reaction temperature after complete addition.

[c] Grignard reagent added to bromobenzene/catalyst mixture. No internal standard in GLPC analysis.

[d] Added at once.

**Claims**

1. A process for producing 3-chloro-2-methyl-[1,1'-biphenyl] characterized by cross-coupling a halobenzene with a 3-chloro 2-methylphenylmagnesium halide in an inert solvent in the presence of a catalytic amount of catalyst selected from palladium metal or nickel (II) acetylacetonate, with the proviso that the catalytic amount of nickel (II) acetylacetonate is greater than 0.01 mole percent, but not more than 0.1 mole percent, based on the 3-chloro-2-methylphenylmagnesium halide.

2. The process according to claim 1 characterized in that the catalyst is palladium metal.

3. The process of claim 2 characterized in that the palladium metal is carried on a solid support.

4. The process of claim 2 characterized in that said solid support is carbon.

5. The process of claim 2 characterized in that said solid support is alumina.

6. The process of claim 2 characterized in that said catalytic amount of palladium metal is 0.02—0.5 mole percent based on 3-chloro-2-methylphenylmagnesium halide.

7. The process according to claim 1 characterized in that the catalyst is nickel (II) acetylacetonate.

8. The process of claim 7 characterized in that said catalytic amount is 0.02—0.04 mole percent.

9. The process of any of claims 1—8 characterized in that the halobenzene is bromobenzene.

10. The process of any of claims 1—9 characterized in that the 3-halo-2-methylphenylmagnesium halide is 3-chloro-2-methylphenylmagnesium chloride.

11. The process of any of claims 1—10 characterized in that the inert solvent is tetrahydrofuran.

**Patentansprüche**

1. Verfahren zur Herstellung von 3-Chlor-2-methyl-[1,1'-biphenyl], gekennzeichnet durch Kreuz-Kupplung eines Halobenzols mit einem 2-Chlor-2-methylphenylmagnesiumhalogenid in einem inerten Lösungsmittel in Gegenwart einer katalytischen Menge eines Katalysators, ausgewählt aus metallischem Palladium oder Nickel (II)-acetylacetonat, mit der Maßgabe, daß die katalytische Menge des Nickel (II)-acetylacetonats größer als 0,01 Mol-%, aber nicht größer als 0,1 Mol-%, bezogen auf das 3-Chlor-2-methylphenylmagnesiumhalogenid ist.

2. Verfahren nach Anspruch 1, dadurch gekennzeichnet, daß der Katalysator metallisches Palladium ist.

3. Verfahren nach Anspruch 2, dadurch gekennzeichnet, daß sich das metallische Palladium auf einem festen Träger befindet.

4. Verfahren nach Anspruch 2, dadurch gekennzeichnet, daß der feste Träger Kohlenstoff ist.

5. Verfahren nach Anspruch 2, dadurch gekennzeichnet, daß der feste Träger Aluminiumoxid ist.

6. Verfahren nach Anspruch 2, dadurch gekennzeichnet, daß die katalytische Menge des metallischen Palladiums 0,02 bis 0,5 Mol-%, bezogen auf das 3-Chlor-2-methylphenylmagnesiumhalogenid beträgt.

7. Verfahren nach Anspruch 1, dadurch gekennzeichnet, daß der Katalysator Nickel (II)-acetylacetonat ist.

8. Verfahren nach Anspruch 7, dadurch gekennzeichnet, daß die katalytische Menge 0,02 bis 0,04 Mol-% beträgt.

9. Verfahren nach einem der Ansprüche 1 bis 8, dadurch gekennzeichnet, daß das Halobenzol Brombenzol ist.

10. Verfahren nach einem der Ansprüche 1 bis 9, dadurch gekennzeichnet, daß das 3-Halo-2-methylphenylmagnesiumhalogenid 3-Chlor-2-methylphenylmagnesiumchlorid ist.

11. Verfahren nach einem der Ansprüche 1 bis 10, dadurch gekennzeichnet, daß das inerte Lösungsmittel Tetrahydrofuran ist.

**Revendications**

1. Procédé de production de 3-chloro-2-méthyl-[1,1'-biphényle] caractérisé en ce qu'on effectue un couplage croisé d'un halobenzène avec un halogénure de 3-chloro-2-méthylphénylmagnésium dans un solvant inerte en présence d'une quantité catalytique d'une quantité de catalyseur choisi entre le palladium métal ou l'acétylacétonate de nickel (II) en spécifiant que la quantité catalytique d'acétylacétonate de nickel (II) est supérieure à 0,01% molaire, mais ne dépasse pas 0,1% molaire, sur la base de l'halogénure de 3-chloro-2-méthylphénylmagnésium.

2. Procédé selon la revendication 1, caractérisé en ce que le catalyseur est le palladium métal.

3. Procédé de la revendication 2, caractérisé en ce que le palladium métal est porté sur un support solide.

4. Procédé de la revendication 2, caractérisé en ce que ledit support solide est le carbone.

5. Procédé de la revendication 2, caractérisé en ce que ledit support solide est l'alumine.

6. Procédé de la revendication 2, caractérisé en ce que ladite quantité catalytique de palladium métal est de 0,02—0,5% molaire sur la base de l'halogénure de 3-chloro-2-méthylphénylmagnésium.

7. Procédé selon la revendication 1, caractérisé en ce que le catalyseur est l'acétylacétonate de nickel (II).

8. Procédé de la revendication 7, caractérisé en ce que ladite quantité catalytique est de 0,02—0,04% molaire.

9. Procédé de l'une quelconque des revendications 1—8 caractérisé en ce que l'halobenzène est le bromobenzène.

10. Procédé de l'une quelconque des revendications 1—9, caractérisé en ce que l'halogénure de 3-halo-2-méthylphénylmagnésium est le chlorure de 3-chloro-2-méthylphénylmagnésium.

11. Procédé de l'une quelconque des revendications 1—10, caractérisé en ce que le solvant inerte est le tétrahydrofuranne.